# EUROPEAN PATENT SPECIFICATION

(11) **EP 2 233 063 B1**
(45) Date of publication and mention of the grant of the patent: **10.02.2016**
(21) Application number: 10002474.4
(22) Date of filing: 10.03.2010
(51) Int. Cl.: A61B 1/005

(54) **Bending instrument for an endoscope and endoscope set**
Instrument de pliage pour endoscope et ensemble endoscope
Biegeinstrument für ein Endoskop und Endoskopsatz

(30) Priority: 24.03.2009 JP 2009071679
(43) Date of publication of application: 29.09.2010
(73) Proprietor: Covidien LP, Mansfield, MA 02048 (US)
(72) Inventor: Funamura, Shigeaki, Shizuoka 437-0004 (JP); Satoh, Takashi, Shizuoka 437-0004 (JP); Yokoyama, Masatoshi, Shizuoka 437-0004 (JP)
(74) Representative: Olsson, Carl H.S.

(56) References cited:
- EP-A1- 0 609 182
- JP-A- 2001 120 496
- US-A- 6 099 464
- US-A1- 2003 014 037

## Description

### Technical Field

The present invention relates to an endoscope set provided with a bending instrument for an endoscope which is used when an endoscope is inserted into a certain internal organ of a patient to observe the inside of that internal organ.

### [Prior Art]

Conventionally, an endoscope is introduced into a patient's stomach, for example, from the nose or mouth so that the stomach wall can be observed. It is necessary in such a case for it to be possible to change the orientation of the tip end of the endoscope so that observations can be made in various directions in order to accurately assess the state of the inside of the stomach. A bending instrument for an endoscope which enables the orientation of the tip end of the endoscope to be changed is therefore used (see JP 2001-120496, for example). This bending sheath for a probe (bending instrument for an endoscope) has a configuration in which a helical cut is provided toward the tip end of a hollow elongate sheath part so as to form an extension, and a tip end holding part is formed at the tip end of the extension.

Furthermore, when an ultrasonic probe is inserted into the bending sheath for a probe and the tip end of the probe makes contact with the inner wall of the tip end holding part, and then the ultrasonic probe continues to be inserted further in, the winding of the extension becomes looser and the extension extends in the axial direction of the sheath. If the ultrasonic probe is further inserted once the extension has stopped extending, the tip end portion of the ultrasonic probe starts to bend away from the axis of the bending sheath. This means that the tip end of the ultrasonic probe can be oriented in the required direction, making it possible to obtain ultrasonic tomographical images for that area.

However, the tip end holding part in the bending sheath for a probe described above is joined to the bending sheath main body by the helical extension, and therefore, if, for example, the endoscope has been inserted into the stomach through a gastrostomy catheter for observation, there is a risk that the extension may not be able to return to its original state when the endoscope is withdrawn after the observation, making withdrawal difficult. Furthermore, if, for example, the body (sliding part) and the tip end holding part (fixed part) of the bending instrument for an endoscope are joined by a deformable cord-like linking part, there is a risk that the tip end holding part will become caught during operation when the linking part bends, which is an impediment to operation.

US Patent Application 2003/0014037 A1 describes a catheter assembly which includes a bendable catheter tube. A sheath is coupled to the bendable catheter such that by sliding movement of the catheter in the sheath, the catheter tube is caused to bow out of an opening in the sheath.

### Summary of the Invention

The present invention provides an endoscope set according to claim 1 comprising an endoscope, an endoscope bending instrument and a gastrostomy catheter.

The present invention has been devised in order to resolve the problems described above, and it aims to provide a bending instrument for an endoscope with which the linking part which joins the sliding part and the fixed part reliably returns to its original state after having bent so that there is no impediment to operation, and furthermore which enables observation of various areas of the internal organs without the endoscope becoming soiled, and an endoscope set which uses the same.

In order to achieve the aims described above, the structural features of the bending instrument for an endoscope according to the present invention lie in the fact that it is a bending instrument for an endoscope which is attached to the tip end of a sheath for covering the endoscope and having a light-transmissive window formed at the tip end thereof, and which causes the tip end portion of the endoscope to bend, said bending instrument for an endoscope being provided with: a fixed part which is attached to the outer periphery at the tip end of the sheath in a state in which it cannot move towards the base end of the sheath; a sliding part which is slidably attached further towards the base end of the sheath than the portion where the fixed part is attached; a flexible linking part for joining the fixed part and the sliding part; and a deformable cylindrical spacer which is fitted on the outer periphery of the sheath between the fixed part and the sliding part.

The bending instrument for an endoscope according to the present invention is a separate member from the sheath, and it is fitted to the tip end of the sheath. This means that the sheath can be configured by a member which can cover at least all the parts of the endoscope which are inserted into the body. The tip end of the sheath which faces the tip end of the endoscope comprises a light-transmissive window, and therefore the sheath causes no reduction in precision during observation within the internal organs using an endoscope. Furthermore, the bending instrument for an endoscope consists of a fixed part which is attached to the tip end of the sheath, a sliding part which is slidably attached further towards the base end of the sheath than the fixed part, a flexible linking part which controls the gap between the fixed part and the sliding part so that it does not exceed a specified length, and a cylindrical spacer which is placed between the fixed part and the sliding part.

Moreover, the linking part in this instance is a cord-like, thread-like, rod-like or narrow sheet-like member, and it is designed to link corresponding parts of the fixed part and sliding part. Furthermore, the whole of the linking part does not need to be flexible - only part of it needs to be so. For example, only the portion of the linking part which is joined to the fixed part and the sliding part may be flexible. Accordingly, as the endoscope is pushed further into the body together with the sheath once the sliding part has been fixed using a specific instrument etc., with the endoscope which is covered by the sheath to which the bending instrument for an endoscope has been attached inserted in the body, the tip ends of the endoscope and sheath are prevented from advancing in the direction of insertion by the fixed part, and they can move together with the fixed part only in the direction of an arc of a circle whose radius is defined by the length of the linking part. Consequently, the position of the tip end of the endoscope changes as it bends together with the sheath.

Here, the cylindrical spacer is deformable and therefore it follows the bending of the endoscope when it deforms. That is to say, this action is possible because corresponding parts of the fixed part and sliding part are joined by the linking part. In this case, the tip ends of the endoscope and the sheath can be oriented in any direction by turning the endoscope in the axial direction together with the sheath, and by adjusting the length of insertion of the endoscope and the sheath. Furthermore, the fixed part is attached to the outer periphery at the tip end of the sheath and therefore there is no interference with observation of the stomach wall etc. by the endoscope. This means that it is possible to change the direction of observation by the endoscope with a simple action, which makes it possible to ascertain the state of the walls of internal organs with greater reliability.

Furthermore, even if the fixed part attempts to retract together with the sheath and the endoscope, and the sliding part attempts to maintain its position when the endoscope is withdrawn from inside the body once observation within the internal organs is complete, the cylindrical spacer is positioned between the fixed part and the sliding part, and therefore the gap between the fixed part and the sliding part does not go below the length of the cylindrical spacer (the contracted length when the spacer is contracted). This means that the linking part does not become twisted, for example becoming tangled with the fixed part, which would be an impediment to operation. That is to say, the length of the cylindrical spacer in the axial direction is set so that the linking part does not twist to an extent that would interfere with operation.

Furthermore, in accordance with the present invention, the endoscope is designed to be bent together with the sheath using the bending instrument for an endoscope, and therefore the endoscope can be made with a simple structure which does not have a mechanism for bending itself. This means that the endoscope has fewer components which might fail. Furthermore, the fixed part may be fixed to the sheath, or it may be detachable from the sheath. Furthermore, in accordance with the present invention, once the insides of the internal organs have been observed, the endoscope can be removed from the patient's body without being soiled by bodily fluids or the like, by pulling out the endoscope from the sheath. As a result, there is almost no need to sterilize or clean the endoscope, which makes sterilizing and cleaning costs almost unnecessary, while the lifespan of the endoscope is extended.

Further structural features of the bending instrument for an endoscope according to the present invention lie in the fact that the cylindrical spacer consists of an elastic member which can extend and contract in the axial direction. This means that the cylindrical spacer can extend and contract in accordance with the gap between the sliding part and the fixed part when the endoscope which is covered by the sheath is pushed into the sliding part of the bending instrument for an endoscope, and therefore the cylindrical spacer does not affect the function of the bending instrument for an endoscope until the cylindrical spacer contracts and acts as a stopper.

Further structural features of the bending instrument for an endoscope according to the present invention lie in the fact that the cylindrical spacer consists of a coil spring member which is longer than the linking part, the spacer being fitted between the fixed part and the linking part in a compressed state. This means that the cylindrical spacer is always urging in the direction of extension of the linking part. If the cylindrical spacer is subjected to a sustained force in the direction of contraction thereof, the cylindrical spacer contracts until it is in a closed state, at which point it starts to act as a stopper. Accordingly, the length of the cylindrical spacer when it has contracted to a closed state is set so that there is no twisting of the linking part to an extent which would interfere with operation, which makes it possible to obtain a suitable bending instrument for an endoscope.

Further structural features of the bending instrument for an endoscope according to the present invention lie in the fact that the cylindrical spacer consists of a coil spring member which is shorter than the linking part, the spacer being fitted between the fixed part and the linking part in a state in which it can slide on the outer periphery of the sheath. In this case, a closed spring which is closed in the axial direction is preferably used as the cylindrical spacer, for example. This means that there is a gap between the sliding part and the cylindrical spacer, and between the fixed part and the cylindrical spacer, or a gap on one side, and therefore, when the endoscope is pulled out from inside the body, the linking part flexes slightly as the sliding part and fixed part come closer together, but the length of the cylindrical spacer is set so that there is no twisting of the linking part to an extent which would interfere with operation, which makes it possible to obtain a suitable bending instrument for an endoscope.

Further structural features of the bending instrument for an endoscope according to the present invention lie in the fact that it is used for insertion of an endoscope into a gastrostomy catheter which has a tubular part formed with an internal through-hole, and a stomach-internal fixed part which is joined to the tip end of the tubular part in a state in which the tip end of the through-hole of the tubular part is open and which is provided with an engaging part in the vicinity of the tip end of the through-hole; the fixed part can pass through the inside of the gastrostomy catheter, and the sliding part can pass through the through-hole of the gastrostomy catheter but engages with the engaging part of the stomach-internal fixed part.

This bending instrument for an endoscope has a structure in which the fixed part can pass through inside the catheter and the sliding part can pass through the through-hole of the gastrostomy catheter, but it engages with the engaging part of the stomach-internal fixed part so as not to be able to pass through the stomach-internal fixed part. Accordingly, when the endoscope which is covered by the sheath to which the bending instrument for an endoscope is attached is inserted into the gastrostomy catheter and the sliding part of the bending instrument for an endoscope reaches the engaging part of the stomach-internal fixed part, the sliding part engages with the engaging part. After this, as the endoscope is pushed further into the gastrostomy catheter together with the sheath, the tip ends of the endoscope and sheath are prevented from advancing in the direction of insertion by the fixed part, and they can move together with the fixed part only in the direction of an arc of a circle whose radius is defined by the length of the linking part.

Furthermore, the portions of the endoscope and the sheath further towards the base end than the tip end are pushed outwards at the tip end of the gastrostomy catheter, and therefore the endoscope projects outwards at the tip end of the gastrostomy catheter while bending together with the sheath. This means that the tip end of the endoscope can be oriented in any direction. It is therefore possible to more reliably ascertain the state of the inner walls of the stomach and to confirm the indwelling position of the gastrostomy catheter, or similar. In this case it is possible to ascertain the direction of the through-hole which opens in the stomach-internal fixed part using the endoscope.

Structural features of the endoscope set according to the present invention lie in the fact that it is provided with any of the bending instruments for an endoscope described above; the endoscope; the sheath; and in addition to this, a gastrostomy catheter which has a tubular part formed with an internal through-hole, and a stomach-internal fixed part which is joined to the tip end of the tubular part in a state in which the tip end of the through-hole of the tubular part is open and which is provided with an engaging part in the vicinity of the tip end of the through-hole, the whole of the bending instrument for an endoscope being inserted into the through-hole, and the sliding part of the bending instrument for an endoscope being made to engage with the engaging part of the stomach-internal fixed part. This means that it is possible to more reliably check the inner walls of the stomach and to confirm the indwelling position of the gastrostomy catheter, and it is possible to obtain an endoscope set with which the used endoscope is not soiled with gastric juices or similar.

### [Brief Description of the Drawings]

[Figure 1] is a front view showing the bending instrument for an endoscope, in accordance with Mode of Embodiment 1 of the present invention;
[Figure 2] is an oblique view of the bending instrument for an endoscope;
[Figure 3] is a front view showing a state in which the bending instrument for an endoscope is fitted to a sheath which covers the endoscope;
[Figure 4] shows the gastrostomy catheter, where (a) is a plan view, (b) is a front view, and (c) is a bottom view;
[Figure 5] is a front view showing the endoscope;
[Figure 6] is a partial cutaway view in cross section showing a state in which the endoscope to which the bending instrument for an endoscope is fitted is positioned above the gastrostomy catheter which is indwelling in the patient;
[Figure 7] is a partial cutaway view in cross section showing a state in which the endoscope to which the bending instrument for an endoscope is fitted is inserted into the gastrostomy catheter which is indwelling in the patient;
[Figure 8] is a partial cutaway view in cross section showing a state in which the inside of the stomach is being checked using the endoscope; and
[Figure 9] is a front view showing the bending instrument for an endoscope, in accordance with Mode of Embodiment 2 of the present invention.

### [Modes of Embodiment of the Invention]

### (Mode of Embodiment 1)

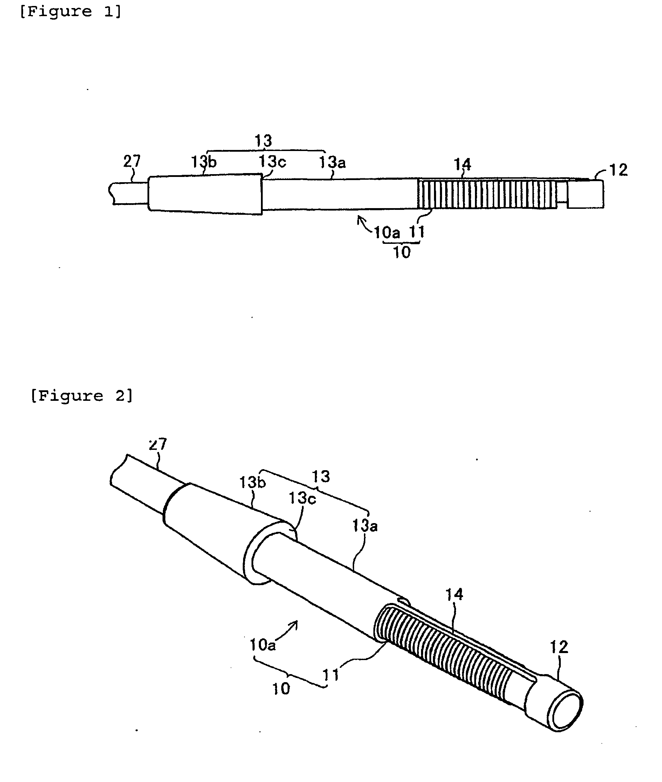

Mode of Embodiment 1 according to the present invention will be described below with reference to the figures. Figures 1 and 2 show a bending instrument 10 for an endoscope, in accordance with this mode of embodiment. This bending instrument for an endoscope 10 consists of a bend-forming member 10a and a cylindrical spacer 11, the bend-forming member 10a comprising a cylindrical fixed part 12, a stepped cylindrical sliding part 13 which is longer in the axial direction than the fixed part 12, and a linear linking part 14 acting as the linking part according to the present invention which joins the fixed part 12 and sliding part 13. The fixed part 12 consists of a tapered cylindrical body in which the diameter of the tip-end portion (the right-hand side in Figures 1 and 2) is somewhat smaller than the diameter of the base-end portion (the left-hand side in Figures 1 and 2). Furthermore, the sliding part 13 consists of a cylindrical insertion part 13a which is positioned at the tip end, and a latch part 13b which is positioned at the base end and whereof the diameter is greater than that of the cylindrical insertion part 13a.

The latch part 13b is formed with a shape that tapers from the tip end towards the base end, and a stepped part 13c which is an annular plane lying perpendicular to the axial direction is formed between the latch part 13b and the cylindrical insertion part 13a. Furthermore, the linear linking part 14 is flexible and it joins the opposing portions at the base end edge of the fixed part 12 and the tip end edge of the cylindrical insertion part 13a of the sliding part 13 in such a way that the fixed part 12 and the sliding part 13 are positioned on the same axis. This linear linking part 14 cannot extend or contract, and a state is maintained such that the distance between the base end edge of the fixed part 12 and the tip end edge of the sliding part 13 does not exceed a fixed length (the length of the linear linking part 14).

Furthermore, the spacer 11 is a closed coil spring comprising a stainless steel wire 0.16 mm in diameter, the length in the axial direction being somewhat shorter than the length of the linear linking part 14. In this mode of embodiment, the length of the linear linking part 14 is set at 13 mm and the length of the spacer 11 is set at 12 mm. Furthermore, the length of the bend-forming member 10a is set at 14 mm. The spacer 11 is a closed spring, i.e. a spring in which each portion in the axial direction is closed so as to form a cylindrical shape, and therefore it can be made to extend by applying tension in the axial direction from a state in which no force is being applied, but it cannot contract even if a pressing force is applied in the axial direction. This spacer 11 is separate from the bend-forming member 10a, and it lies between the fixed part 12 and sliding part 13 during use.

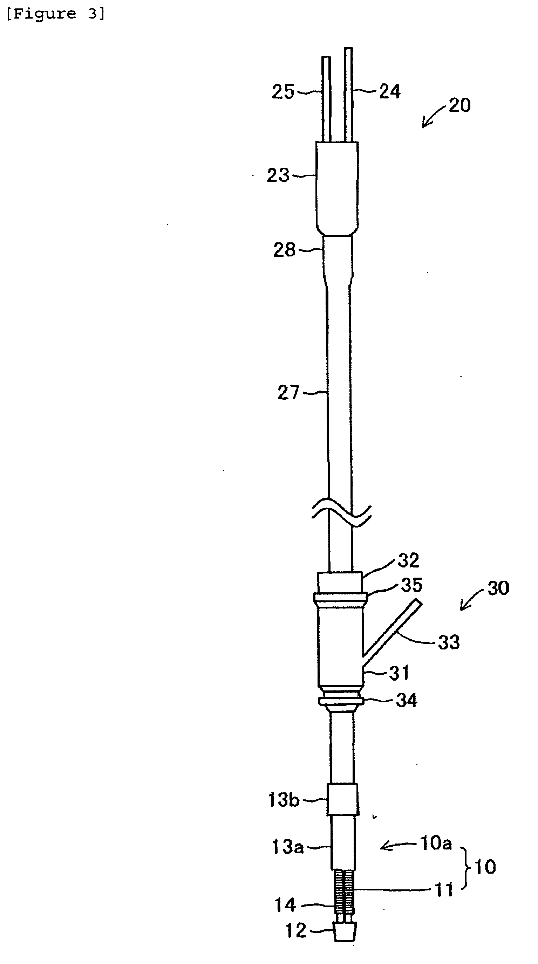

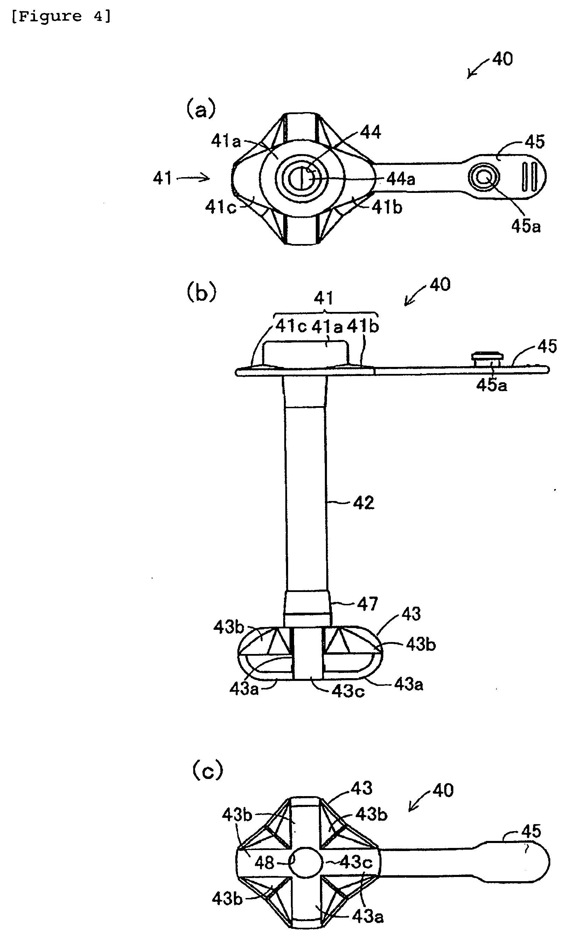

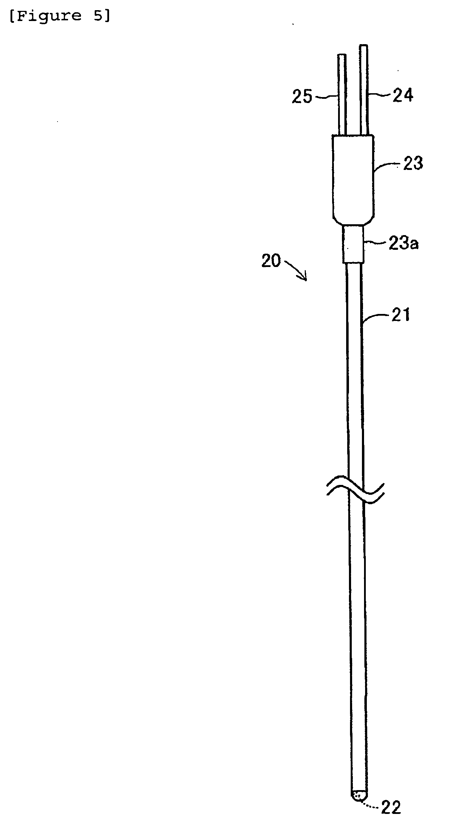

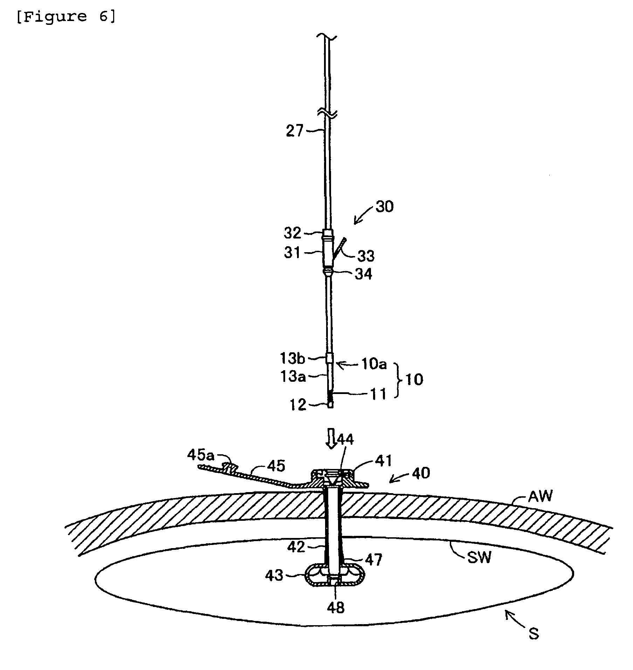

As shown in Figure 3, the bending instrument 10 for an endoscope configured in the manner described above is assembled with a sheath 27 for covering the endoscope 20; it is inserted into the gastrostomy catheter 40 shown in Figure 4 using an insertion aid 30, for example. The endoscope set according to the present invention consists of the bending instrument for an endoscope 10, the endoscope 20, the sheath 27 and the gastrostomy catheter 40, and it is possible to observe the inside of a patient's stomach S (see Figures 6 to 8) using this endoscope set. As shown in Figure 5, the endoscope 20 has a configuration in which a lens 22 is attached to the tip end of a fibrescope shaft 21 and a connecting part 23 is attached to the rear end thereof.

The fibrescope shaft 21 is flexible and it consists of a bundle of fibres comprising a number of light guides (not depicted) for irradiating light, and an image guide (not depicted) for sending reflected light via the lens 22. The connecting part 23 supports wiring 24 for connecting the image guide to an image display device (not depicted), and wiring 25 for connecting the light guides to a light source device (not depicted), with a gap between the wiring. The lens 22 sends images obtained by light irradiation to the image display device, via the image guide and the wiring 24. The image display device then enlarges the images based on the reflected light sent and displays them on an image display part provided in the image display device.

The sheath 27 is flexible and consists of a tube which is closed off by a window (not depicted) wherein the tip end is light-transmissive, and in which the base end 28 on the open side is somewhat greater in diameter than the other portion. Said sheath 27 is formed to have a thickness which allows it to cover the fibrescope shaft 21, and it is fitted to the fibrescope shaft 21 by pushing a narrow-diameter part 23a at the tip end of the connecting part 23 into the base end 28. The sheath 27 is then retained in the fibrescope shaft 21 using a member such as a clamp, a fastening tool or a clasping tool, and in this state a configuration is adopted in which the lens 22 is in contact with the inner surface of the window.

Furthermore, the outer diameter of the sheath 27 is somewhat smaller than the inner diameter of the base end portion of the fixed part 12 and the inner diameter of the sliding part 13 in the bend-forming member 10a. Consequently, when the sheath 27 is inserted from its tip end into the fixed part 12 from the base end towards the tip end thereof, the outer periphery of the tip end of the sheath 27 comes into contact with the inner peripheral part at the tip end of the fixed part 12. Consequently, the sheath 27 cannot pass through inside the fixed part 12 in a state in which the fibrescope shaft 21 is covered. Moreover, when the bending instrument 10 for an endoscope is being used, the fixed part 12 is fixed to the outer periphery of the tip end of the sheath 27 by means of adhesive, solvent welding or heat welding. Furthermore, when the sheath 27 has been inserted into the sliding part 13, the sliding part 13 can slide in the longitudinal direction of the sheath 27.

The insertion aid 30 is an instrument which is fitted to the gastrostomy catheter 40 to smooth the passage of the endoscope 20 through the gastrostomy catheter 40, and the main body thereof consists of a cylindrical main body 31, a valve restraining member 32, and a branch pipe 33 which branches off from the cylindrical main body 31, as shown in Figure 3. An annular deformable sealing member (not depicted) is placed between the cylindrical main body 31 and the valve restraining member 32. The cylindrical main body 31 is formed as an elongate cylinder which allows the fibrescope shaft 21 to pass through together with the sheath 27. Furthermore, a connecting part 34 is formed at the tip end of the cylindrical main body 31, and an insertion opening 35 is formed at the base end of the cylindrical main body 31.

The branch pipe 33 is formed with a cylindrical shape extending obliquely upwards at an inclination of approximately 45° to the cylindrical main body 31 from the upper side of the connecting part 34 of the cylindrical main body 31, and it has a narrower diameter than the cylindrical main body 31. Air is supplied from an air supply device (not depicted) to this branch pipe 33, and the air supplied is sent into the cylindrical main body 31. Furthermore, an airflow channel (not depicted) is formed for the passage of air between the inside of the cylindrical main body 31 and the lower end inside the connecting part 34, and air which is sent to the lower end inside the cylindrical main body 31 is discharged to the outside from the lower end of the connecting part 34.

The gastrostomy catheter 40 comprises an external fixed part 41, a tubular part 42 which is linked to the centre of the lower end surface of the external fixed part 41, and a stomach-internal fixed part 43 which is attached to the lower end of the tubular part 42, all these components being made of a soft plastic material such as polyurethane or silicone. In the description that follows, the external fixed part 41 will be taken as the upper side, and the stomach-internal fixed part 43 will be taken as the lower side. The external fixed part 41 comprises an insertion opening 41a which is annular and fairly thick, and projecting pieces 41b, 41c which project at both sides from the lower end of both side parts of the insertion opening 41a. The function of these projecting pieces 41b, 41c is to prevent the gastrostomy catheter 40 which is arranged in a gastric fistula in a patient from being pulled into the stomach S.

A valve body 44a which is formed with a central slit is then provided on the inner peripheral surface of an insertion hole 44 which is formed in the centre of the insertion opening 41a, passing through vertically. A strip-like cover part 45 which is provided with a stopper 45a for closing off the insertion hole 44 of the insertion opening 41a is linked to the tip end of the projecting piece 41b. The tubular part 42 has a cylindrical shape, and a through-hole (not depicted) is formed inside it in order to allow the passage of fluids such as nutrients and food in fluid form, the upper end of the through-hole communicating with the insertion hole 44 of the external fixed part 41.

The stomach-internal fixed part 43 is connected to the tubular part 42 by way of a cylindrical connecting part 47 which is fixed to the lower end of the tubular part 42. The stomach-internal fixed part 43 comprises four strip-shaped linking parts 43a which are linked to the edge of a lower end opening of the connecting part 47 and extend in four directions, four linking film parts 43b which are provided between the upper parts of each of the linking parts 43a and form a roughly dome-shaped stomach wall contact part with the four linking parts 43a, and a converging part 43c where the tip ends of all of the linking parts 43a converge. The four linking parts 43a comprise strip-like members which are bent into substantially semi-circular shapes which split into four directions from the lower end of the connecting part 47, respectively extending downwards from the horizontal, after which they converge below the central axis of the tubular part 42, linking to form the converging part 43c.

Furthermore, the stomach-internal fixed part 43 is made of a soft, flexible elastic material, and the overall flat roughly spherical shape is normally maintained by means of this elasticity, as shown in Figure 4(b), but the shape can be extended to make it straight and elongate by pulling the converging part 43c downwards. The stomach-internal fixed part 43 can pass through the gastric fistula in the patient when it is in this straight and elongate state. Furthermore, the lower end of the through-hole of the tubular part 42 opens between the upper ends of the linking parts 43a. Then, spaces formed between the lower parts of each of the linking parts 43a form channels for the passage of fluids such as nutrients and food in fluid form sent out from the through-hole of the tubular part 42 into the stomach S.

A through-hole 48 is additionally formed in the centre of the converging part 43c. The diameter of the through-hole 48 is smaller than the diameter of the insertion hole 44 in the external fixed part 41 and the through-hole of the tubular part 42, and the engaging part according to the present invention comprises the peripheral edge at the upper end of this through-hole 48. The stomach-internal fixed part 43 configured in this manner is positioned on the inner surface of the patient's stomach wall SW (see Figures 6 to 8) and its function is to prevent the gastrostomy catheter 40 from being removed from the patient's body.

The outer diameter of the fixed part 12 and the cylindrical insertion part 13a of the sliding part 13 on the bend-forming member 10a is smaller than the diameter of the through-hole 48 of the stomach-internal fixed part 43. The outer diameter of the stepped part 13c of the sliding part 13 is smaller than the diameter of the insertion hole 44 of the external fixed part 41 and of the through-hole of the tubular part 42, but greater than the diameter of the through-hole 48 of the stomach-internal fixed part 43. Consequently, the fixed part 12 and the cylindrical insertion part 13a of the sliding part 13 can penetrate into the gastrostomy catheter 40 from the insertion hole 44 towards the through-hole 48, but the stepped part 13c (latch part 13b) of the sliding part 13 engages with the peripheral edge at the upper end of the through-hole 48 and is unable to pass through the through-hole 48.

A description will be given next of the method of confirming the indwelling position of the gastrostomy catheter 40 while also observing the inside of the stomach S using the endoscope set configured in the manner described above. Figure 6 shows a state in which the gastrostomy catheter 40 is indwelling in a gastric fistula provided in the abdominal wall AW and the stomach wall SW of a patient. In this state, the stopper 45a of the gastrostomy catheter 40 is removed from the insertion hole 44 to open the upper end of the insertion hole 44. Furthermore, the fibrescope shaft 21 of the endoscope 20 is covered by the sheath 27, and the bending instrument 10 for an endoscope and the insertion aid 30 are attached to the sheath 27. The endoscope 20 etc. which are in this state are moved down in the direction of the arrow shown in the figures so that the fibrescope shaft 21 is inserted into the insertion hole 44 of the gastrostomy catheter 40 together with the sheath 27 and the bending instrument 10 for an endoscope, and the insertion aid 30 is pushed into the gastrostomy catheter 40.

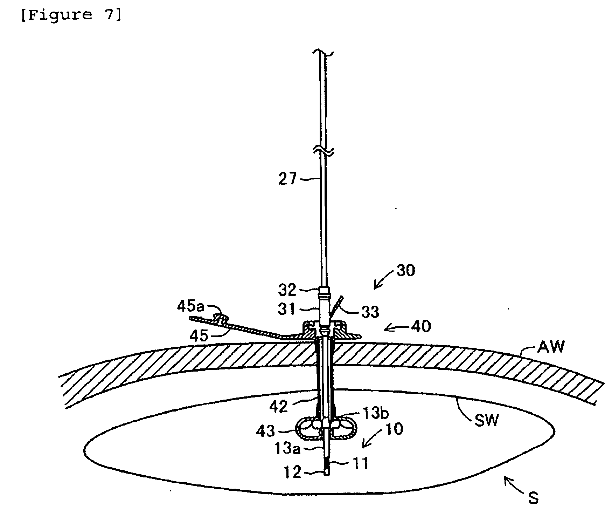

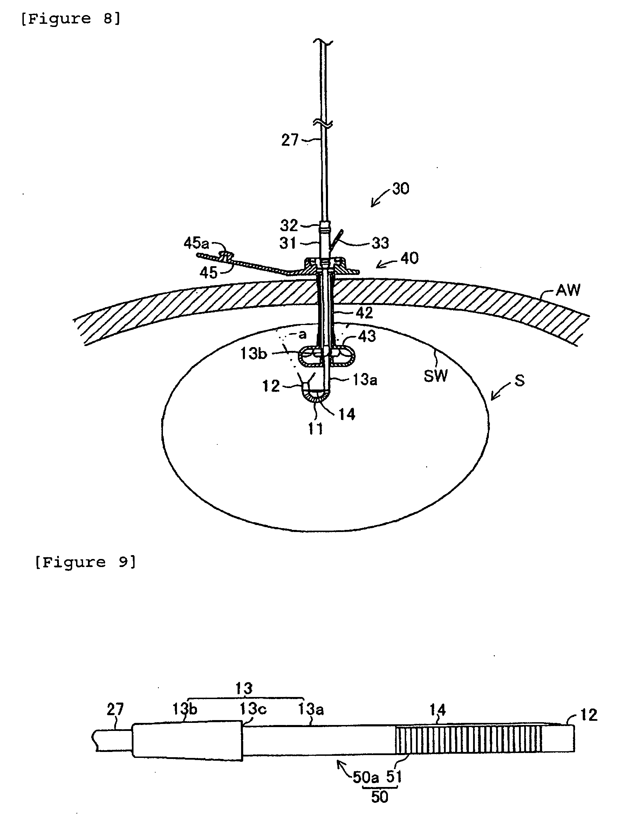

This makes it possible for the connecting part 34 of the insertion aid 30 to engage with the inner peripheral surface of the insertion hole 44 of the gastrostomy catheter 40, the insertion aid 30 and the gastrostomy catheter 40 then reaching the state shown in Figure 7 in which they are linked in an airtight manner. The endoscope 20 is then further inserted towards the lower side of the gastrostomy catheter 40 together with the sheath 27 and the bending instrument 10 for an endoscope, and the lower portion of the fibrescope shaft 21, sheath 27 and bending instrument 10 for an endoscope project downwards from the through-hole 48 of the gastrostomy catheter 40. Next, air is supplied from the air supply device to inside the branch pipe 33, and this air is sent into the stomach S from the connecting part 34 via the tubular part 42 of the gastrostomy catheter 40. This allows the stomach S to expand, as shown in Figure 8.

In this state, light is generated by means of the light source device, this light being irradiated towards the stomach wall SW. Furthermore, in this case, the endoscope 20 and the sheath 27 etc. are pushed into the body as required, whereby the lower portion of the fibrescope shaft 21 bends together with the sheath 27 so that it is possible to change the position of irradiation of the stomach wall SW. That is to say, from the state shown in Figure 7, when the fibrescope shaft 21 and the sheath 27 are pushed further in with respect to the bending instrument 10 for an endoscope, the fixed part 12 of the bending instrument 10 for an endoscope and the lower portion of the sliding part 13 pass through the through-hole 48 of the stomach-internal fixed part 43 and enter the stomach S, but the engaging part 13b of the sliding part 13 engages with the peripheral edge at the upper end of the through-hole 48 and is held in the stomach-internal fixed part 43.

Consequently, the fixed part 12 moves so as to describe the arc of a circle, with the linear linking part 14 as the radius, and the tip end portion of the fibrescope shaft 21 and sheath 27 and the spacer 11 follow this movement of the fixed part 12 while bending, and protrude inside the stomach S. The range shown by the two-dot chain line a in Figure 8 shows the range of light irradiation by the light guides. Light which is irradiated by means of the light guides and reflected off the stomach wall SW is focused by the lens 22, after which it is sent to the image display device by way of the image guide and the wiring 24 of the fibrescope shaft 21. By means of this, it is possible to check the state of the inside of the stomach S, or to ascertain whether or not the stomach-internal fixed part 43 of the gastrostomy catheter 40 is correctly placed inside the stomach S, or carry out other operations.

After the inside of the stomach S has been observed using the endoscope 20, an operation is carried out to remove the endoscope 20 from the gastrostomy catheter 40 together with the sheath 27, bending instrument 10 for an endoscope and insertion aid 30, and also to remove the sheath 27 and bending instrument 10 for an endoscope from the fibrescope shaft 21. In this operation, the endoscope 20 is first of all pulled upwards slightly together with the bending instrument 10 for an endoscope. At this point, if the fixed part 12 attempts to retract inside the gastrostomy catheter 40 together with the fibrescope shaft 21 and sheath 27, and the sliding part 13 does not move with respect to the gastrostomy catheter 40, the linear linking part 14 will become twisted.

However, the spacer 11 is fitted between the fixed part 12 and the sliding part 13 on the outer peripheral surface of the sheath 27, and this spacer 11 is slightly shorter than the linear linking part 14. Accordingly, the gap between the fixed part 12 and the sliding part 13 is maintained by the spacer to the extent that the linear linking part 14 only twists slightly. This means that there are no longer situations where the fixed part 12 becomes tangled with the linear linking part 14 so that the bending instrument 10 for an endoscope cannot be removed from the gastrostomy catheter 40. Then, when the state shown in Figure 7 has been reached, the engagement between the connecting part 34 of the insertion aid 30 and the inner peripheral surface of the insertion hole 44 of the gastrostomy catheter 40 is released, and the endoscope 20 is pulled upwards together with the bending instrument 10 for an endoscope and the insertion aid 30, whereby they are withdrawn from the gastrostomy catheter 40.

In addition, after the insertion aid 30 has been removed from the sheath 27, the fibrescope shaft 21 is withdrawn from the sheath 27. The bending instrument 10 for an endoscope is then discarded together with the sheath 27, and the endoscope can be used on a subsequent occasion. At this point, the fibrescope shaft 21 and the lens 22 of the endoscope 20 do not come into contact with the liquids and residues inside the patient's body and stomach S, so they are not soiled and there is no need for the most part to clean or sterilize them. Furthermore, when the endoscope 20 is reused, a new sheath 27 and a new bending instrument 10 for an endoscope are used. Furthermore, when nutrient fluid is supplied to the patient's stomach S, for example, by way of the gastrostomy catheter 40 which is indwelling in the patient's body, a connector for a tube extending from a container housing the nutrients is connected to the insertion hole 44 of the gastrostomy catheter 40. Then, after use, the insertion hole 44 is closed by the stopper 45a.

In this way, with the bending instrument 10 for an endoscope according to this mode of embodiment, the whole of the fibrescope shaft 21 of the endoscope 20 can be covered by the sheath 27, and therefore the endoscope 20 does not become soiled by bodily fluids and the like. As a result, there is almost no need to sterilize or clean the endoscope, which makes sterilizing and cleaning costs almost unnecessary, while the lifespan of the endoscope is extended. Furthermore, when the endoscope 20 which is covered by the sheath 27 to which the bending instrument 10 for an endoscope has been fitted is inserted from the insertion hole 44 of the gastrostomy catheter 40, and the latch part 13b of the sliding part 13 engages with the peripheral edge at the upper end of the through-hole 48, after which the endoscope 20 is inserted, the tip ends of the endoscope 20 and sheath 27 can be bent to orient them in various directions. Consequently, it is possible to observe all areas inside the stomach S. At this point, the spacer 11 deforms as the endoscope 20 bends, and therefore the function of the bending instrument 10 for an endoscope is not adversely affected.

Furthermore, when the endoscope 20 is withdrawn from the gastrostomy catheter 20 once the inside of the stomach S has been observed, the spacer 11 is positioned between the fixed part 12 and sliding part 13, and therefore the gap between the fixed part 12 and the sliding part 13 does not become less than the length of the spacer 11. This means that situations do not arise where the linear linking part 14 becomes twisted, the fixed part 12 becomes entangled, or the through-hole 48 becomes blocked so that they cannot be withdrawn from the gastrostomy catheter 40. Furthermore, according to this mode of embodiment, the endoscope 20 is designed to bend together with the sheath 27 by use of the bending instrument 10 for an endoscope, and therefore the endoscope 20 can be made with a simple structure which does not have a mechanism for bending itself. This means that the endoscope 20 has fewer components which might fail.

### (Mode of Embodiment 2)

Figure 9 shows a bending instrument 50 for an endoscope according to Mode of Embodiment 2 of the present invention. This bending instrument 50 for an endoscope consists of a bend-forming member 50a and a spacer 51, the bend-forming member 50a having the same structure as the bend-forming member 10a described above. Accordingly, similar components bear similar reference numbers and a description of them will be omitted. Furthermore, the spacer 51 comprises a coil compression spring of which the length, in an unstressed state, is set at 14 mm. The spacer 51 is placed between the fixed part 12 and the sliding part 13 in a compressed state when it is being used. This bending instrument 50 for an endoscope is fitted to the sheath 27 described above, and it is used to bend the tip end of the endoscope 20.

According to this bending instrument 50 for an endoscope, the spacer 51 is designed to always urge the bend-forming member 50a in the direction of extension of the linear linking part 14. If the spacer 51 is subjected to a sustained force in the direction of contraction thereof, the spacer 51 contracts until it is in a closed state, at which point it starts to act as a stopper. Accordingly, the length of the spacer 51 when it has contracted to a closed state is set so that there is no twisting of the linking part 14 to an extent which would interfere with operation, which makes it possible to obtain a suitable bending instrument 50 for an endoscope. The other operational effects of the bending instrument 50 for an endoscope are the same as those of the bending instrument 10 for an endoscope described above.

Furthermore, the bending instrument according to the present invention is not limited to the modes of embodiment described above, and suitable modifications may be made without departing from the technical scope of the present invention. For example, in the modes of embodiment described above, the bending instruments 10, 50 for an endoscope are fitted, along with the sheath 27, to the endoscope 20 for observing the inside of the stomach S, but these instruments may be fitted to endoscopes for observing areas or internal organs inside the body other than just the stomach S. In this case, the gastrostomy catheter 40 and insertion aid 30 may be dispensed with. An instrument provided with an engaging part for preventing the advance of the sliding part is used instead of the gastrostomy catheter 40, depending on the area of the body which is being observed. Suitable modifications may also be made to the structures of the bending instrument for an endoscope, endoscope, sheath and gastrostomy catheter, without departing from the technical scope of the present invention.

### [Key to Symbols]

10, 50...bending instrument for endoscope; 11, 51...spacer; 12...fixed part; 13...sliding part; 14...linear linking part; 20...endoscope; 27...sheath; 40...gastrostomy catheter; 42...tubular part; 43...stomach-internal fixed part; 44...insertion hole; 48...through-hole; AW... abdominal wall; SW...stomach wall.

## Claims

1. An endoscope set comprising:
an endoscope;
a sheath (27) for covering the endoscope, the sheath having a light-transmissive window formed at the tip end thereof,
a bending instrument (10) attached to the tip end of the sheath for causing in use the tip end portion of the endoscope to bend,
said bending instrument for an endoscope being **characterized in that** the bending instrument is provided with:
a fixed part (12) which is fixedly attached to the outer periphery of the sheath at the tip end thereof such that the fixed part cannot move towards the base end of the abovementioned sheath;
a sliding part (13) which is slidably attached further towards the base end of the abovementioned sheath than the portion where the abovementioned fixed part is attached;
a flexible linking part (14) for joining the abovementioned fixed part and the abovementioned sliding part; and
a deformable cylindrical spacer (11) which is fitted on the outer periphery of the abovementioned sheath between the abovementioned fixed part and the abovementioned sliding part; and
a gastrostomy catheter (40) which has a tubular part (42) formed with an internal through-hole, and a part (43) adapted to be fixed to the inner side of the stomach which is joined to the tip end of the abovementioned tubular part in a state in which the tip end of the through-hole of the abovementioned tubular part is open and which is provided with an engaging part in the vicinity of the tip end of the abovementioned through-hole, the whole of the abovementioned bending instrument for an endoscope being insertable into the abovementioned through-hole, and the sliding part of the abovementioned bending instrument for an endoscope being engageable with the engaging part of the abovementioned stomach-internal fixed part (43) adapted to be fixed to the inner side of the stomach.

2. The endoscope set according to Claim 1, in which the bending instrument cylindrical spacer (11) consists of an elastic member which can extend and contract in the axial direction.

3. The endoscope set according to Claim 1 or 2, in which the bending instrument cylindrical spacer (11) consists of a coil spring member which is longer than the abovementioned linking part (14), the spacer being fitted between the abovementioned fixed part (12) and the abovementioned linking part (14) in a compressed state.

4. The endoscope set according to Claim 1 or 2, in which the bending instrument cylindrical spacer (11) consists of a coil spring member which is shorter than the abovementioned linking part (14), the spacer being fitted between the abovementioned fixed part (12) and the abovementioned linking part (14) in a state in which it can slide on the outer periphery of the abovementioned sheath.

5. The endoscope set according to any one of Claims 1 to 4, wherein the abovementioned bending instrument fixed part (12) can pass through the inside of the abovementioned gastrostomy catheter (40), and the abovementioned sliding part (13) can pass through the through-hole of the abovementioned gastrostomy catheter but engages with the engaging part of the abovementioned part (43) adapted to be fixed to the inner side of the stomach.

## Patentansprüche

1. Endoskopsatz, umfassend:
ein Endoskop;
eine Hülle (27) zur Abdeckung des Endoskops, wobei die Hülle ein an ihrem Spitzenende geformtes lichtdurchlässiges Fenster aufweist,
ein Biegeinstrument (10), das am Spitzenende der Hülle befestigt ist, um zu veranlassen, dass sich der Spitzenendabschnitt des Endoskops im Gebrauch biegt, wobei das Biegeinstrument für ein Endoskop **dadurch gekennzeichnet ist, dass** das Biegeinstrument Folgendes aufweist:
ein fixiertes Teil (12), das fest an dem Außenumfang der Hülle an deren Spitzenende befestigt ist, so dass sich das fixierte Teil nicht zum Basisende der oben erwähnten Hülle bewegen kann;
ein Gleitteil (13), das weiter zum Basisende der oben erwähnten Hülle hin verschiebbar befestigt ist als der Abschnitt, an dem das oben erwähnte fixierte Teil befestigt ist;
ein flexibles Verbindungsteil (14) zur Verbindung des oben erwähnten fixierten Teils und des oben erwähnten Gleitteils; und
einen verformbaren zylinderförmigen Abstandshalter (11), der auf dem Außenumfang der oben erwähnten Hülle zwischen dem oben erwähnten fixierten Teil und dem oben erwähnten Gleitteil angebracht ist; und
einen Gastrostomie-Katheter (40), der ein schlauchförmiges Teil (42) aufweist, das mit einer inneren Durchgangsbohrung geformt ist, und ein Teil (43), das ausgelegt ist, an der Innenseite des Magens fixiert zu werden, und das mit dem Spitzenende des oben erwähnten schlauchförmigen Teils in einem Zustand befestigt ist, in dem das Spitzenende der Durchgangsbohrung des oben erwähnten schlauchförmigen Teils offen ist, und das mit einem Eingriffsteil in der Umgebung des Spitzenendes der oben erwähnten Durchgangsbohrung ausgestattet ist, wobei das ganze oben erwähnte Biegeinstrument für ein Endoskop in die oben erwähnte Durchgangsbohrung eingeführt werden kann, und das Gleitteil des oben erwähnten Biegeinstruments für ein Endoskop mit dem Eingriffsteil des oben erwähnten, im Mageninneren fixierten Teils (43) in Eingriff gebracht werden kann.

2. Endoskopsatz nach Anspruch 1, wobei der zylinderförmige Abstandshalter (11) des Biegeinstruments aus einem elastischen Element besteht, das sich in die axiale Richtung ausdehnen und zusammenziehen kann.

3. Endoskopsatz nach Anspruch 1 oder 2, wobei der zylinderförmige Abstandshalter (11) des Biegeinstruments aus einem Spiralfederelement besteht, das länger als das oben erwähnte Verbindungsteil (14) ist, wobei der Abstandshalter zwischen dem oben erwähnten fixierten Teil (12) und dem oben erwähnten Verbindungsteil (14) in einem komprimierten Zustand angebracht ist.

4. Endoskopsatz nach Anspruch 1 oder 2, wobei der zylinderförmige Abstandshalter (11) des Biegeinstruments aus einem Spiralfederelement besteht, das kürzer als das oben erwähnte Verbindungsteil (14) ist, wobei der Abstandshalter zwischen dem oben erwähnten fixierten Teil (12) und dem oben erwähnten Verbindungsteil (14) in einem Zustand angebracht ist, in dem er auf dem Außenumfang der oben erwähnten Hülle gleiten kann.

5. Endoskopsatz nach einem der Ansprüche 1 bis 4, wobei das oben erwähnte fixierte Teil (12) des Biegeinstruments durch die Innenseite des oben erwähnten Gastrostomie-Katheters (40) verlaufen kann, und das oben erwähnte Gleitteil (13) durch die Durchgangsbohrung des oben erwähnten Gastrostomie-Katheters verlaufen kann, aber mit dem Eingriffsteil des oben erwähnten Teils (43) in Eingriff kommt.

## Revendications

1. Ensemble endoscope comprenant :
un endoscope ;
une gaine (27) servant à recouvrir l'endoscope, la gaine comportant une fenêtre translucide formée au niveau de son extrémité terminale,
un instrument de flexion (10) attaché à l'extrémité terminale de la gaine et servant, lors de l'utilisation, à amener la partie d'extrémité terminale de l'endoscope à fléchir,
ledit instrument de flexion pour un endoscope étant **caractérisé en ce que** l'instrument de flexion comprend :
une partie fixe (12) qui est attachée de manière fixe à la périphérie extérieure de la gaine au niveau de son extrémité terminale de telle sorte que la partie fixe ne puisse se déplacer vers l'extrémité de base de ladite gaine ;
une partie coulissante (13) qui est attachée à coulissement plus près de l'extrémité de base de ladite gaine que la partie à laquelle est attachée ladite partie fixe ;
une partie de liaison souple (14) servant à relier ladite partie fixe et ladite partie coulissante ; et
une entretoise cylindrique déformable (11) qui est ajustée sur la périphérie extérieure de ladite gaine entre ladite partie fixe et ladite partie coulissante ; et
un cathéter de gastrostomie (40) qui comporte une partie tubulaire (42) formée de façon à comprendre un trou traversant intérieur, et une partie (43) conçue pour être fixée au côté intérieur de l'estomac qui est reliée à l'extrémité terminale de ladite partie tubulaire dans une configuration dans laquelle l'extrémité terminale du trou traversant de ladite partie tubulaire est ouverte et qui comprend une partie d'accouplement à proximité de l'extrémité terminale dudit trou traversant, l'intégralité dudit instrument de flexion pour un endoscope pouvant être insérée dans ledit trou traversant, et la partie coulissante dudit instrument de flexion pour un endoscope pouvant être accouplée avec la partie d'accouplement de ladite partie (43) fixe à l'intérieur de l'estomac conçue pour être fixée au côté intérieur de l'estomac.

2. Ensemble endoscope selon la revendication 1, dans lequel l'entretoise cylindrique (11) de l'instrument de flexion est constituée d'un élément élastique qui peut s'étendre et se contracter dans la direction axiale.

3. Ensemble endoscope selon la revendication 1 ou 2, dans lequel l'entretoise cylindrique (11) de l'instrument de flexion est constituée d'un élément formant ressort hélicoïdal qui est plus long que ladite partie de liaison (14), l'entretoise étant ajustée entre ladite partie fixe (12) et ladite partie de liaison (14) dans un état comprimé.

4. Ensemble endoscope selon la revendication 1 ou 2, dans lequel l'entretoise cylindrique (11) de l'instrument de flexion est constituée d'un élément formant ressort hélicoïdal qui est plus court que ladite partie de liaison (14), l'entretoise étant ajustée entre ladite partie fixe (12) et ladite partie de liaison (14) dans une configuration dans laquelle elle peut coulisser sur la périphérie extérieure de ladite gaine.

5. Ensemble endoscope selon l'une quelconque des revendications 1 à 4, dans lequel ladite partie fixe (12) de l'instrument de flexion peut passer à travers l'intérieur dudit cathéter de gastrostomie (40), et ladite partie coulissante (13) peut passer à travers le trou traversant dudit cathéter de gastrostomie mais s'accouple avec la partie d'accouplement de ladite partie (43) conçue pour être fixée sur le côté intérieur de l'estomac.
